# EUROPEAN PATENT APPLICATION

(11) **EP 1 236 461 A1**
(43) Date of publication of application: **04.09.2002**
(21) Application number: 00979952.9
(22) Date of filing: 07.12.2000
(51) Int. Cl.: A61K 7/28

(54) **TOOTHPASTE COMPOSITIONS**

(30) Priority: 08.12.1999 JP 37635399; 11.10.2000 JP 2000310936
(71) Applicant: Lion Corporation, Tokyo 130-8644 (JP)
(72) Inventor: SANO, Hiroshi, Lion Corporation, Sumida-ku, Tokyo 130-8644 (JP); NISHINAGA, Eiji, Lion Corporation, Sumida-ku, Tokyo 130-8644 (JP); KIKUCHI, Yasuo, Lion Corporation, Sumida-ku, Tokyo 130-8644 (JP); YOSHIMURA, Masaki, Lion Corporation, Sumida-ku, Tokyo 130-8644 (JP)
(74) Representative: Casalonga, Axel
(86) International application number: JP0008636
(87) International publication number: WO01041726

(57) **Abstract**

The invention provides a dentifrice composition, which comprises an enzyme and/or an antiplasmin, and an anionic surface active agent, characterized by further comprising a hydroxyethyl cellulose·dimethyldiallylammonium salt, and one or more flavor components (A) selected from the group consisting of anethole, carvone, cineol, methyl salicylate, eugenol, ethyl butylate and cinnamic aldehyde and 1-menthol (B) being contained at a ratio by weight of 1:9 to 8:2.

## Description

### TECHNICAL FIELD

This invention relates to a dentifrice composition which is improved in intraoral retention of an enzyme or an antiplasmin agent, foaming property and storage stability. More particularly, the invention relates to a dentifrice composition comprising an enzyme and/or an antiplasmin agent, and an anionic surface active agent wherein a specific type of flavor component such as anethole or the like and 1-menthol are further formulated at a specific ratio along with a specific type of cationic polymer, so that the intraoral retention of the enzyme and/or the antiplasmin agent is enhanced and the composition is foamed well upon use without causing syneresis upon storage.

### BACKGROUND ART

Hitherto, dentifrices comprising a dextranase or a mutanase as an effective ingredient are known as ones having a high tartar formation-suppressing effect, and dentifrices comprising lysozyme as an effective ingredient are also known as ones having a high gingivitis suppressing effect. In this technical field, techniques of improving the intraoral retention of these enzymes have been under development.

It is known that the antiplasmin agent-containing dentifrice is effective in controlling antiphlogosis and hemorrhage and has a good effect of preventing a periodental disease. In order to enhance the preventive effect of the periodental disease, the development of a technique of improving the intraoral retention of an antiplasmin agent in the dentifrice has been continued.

One of techniques of improving the retention of effective ingredients on a mucosal membrane in the mouth and the surfaces of teeth includes a method of utilizing a cationic polymer. For instance, techniques of using a fluorine compound serving as an effective ingredient and a cationic polymer in combination are disclosed (Japanese patent Laid-open Nos. Sho 62-145010 and Hei 6-65035).

In this connection, however, an anionic surface active agent is usually employed as a foaming agent in an enzyme and/or antiplasmin agent-containing dentifrice composition, under which if the cationic polymer disclosed in the above-mentioned known references is added to, a complex material of the cationic polymer and the anionic surface active agent is formed. This material serves to worsen the intraoral retention of the enzyme and antiplasmin agent and also the foaming property and storage stability of the dentifrice composition. This places limitation on the use of an anionic surface active agent, and proposal of a technical solution therefor has been desired.

Further, the use of cationic polymers has the substantial problem of suppressing foaming upon brushing, and proposal of a method of technically solving this problem has been expected.

### DISCLOSURE OF INVENTION

The object of the invention is to provide a dentifrice composition which enhances the intraoral retention of an enzyme and an antiplasmin agent and has excellent properties such as a good foaming property upon use and no tendency toward syneresis during storage.

The present inventors have made intensive studies in order to attain the above object, and as a result, found that while a hydroxyethyl cellulose·dimethyldiallylammonium salt that is a cationic polymer is formulated in a system where an enzyme and/or an antiplasmin agent and an anionic surface active agent co-exist, a specific type of flavor component such as anethole or the like and 1-menthol are further formulated at a specific ratio, whereby the intraoral retention of the enzyme and the antiplasmin agent is enhanced, and the foaming property in use and storage stability are improved. The invention has been accomplished based on this finding.

More particularly, according to the invention, there is provided a dentifrice composition of the type which comprises an enzyme and/or an antiplasmin agent and an anionic surface active agent, wherein a hydroxyethyl cellulose·dimethyldiallylammonium salt is contained, and one or more flavor components (A) selected from anethole, carvone, cineol, methyl salicylate, eugenol, ethyl butylate and cinnamic aldehyde and 1-menthol (B) are contained at a ratio by weight of 1:9 to 8:2.

### BEST MODE FOR CARRYING OUT THE INVENTION

The dentifrice composition of the invention comprises an anionic surface active agent along with an enzyme and/or an antiplasmin agent serving as an effective ingredient.

Examples of the enzymes include dextranase, mutanase, amylase, lysozyme, protease, bacteriolysin or the like, and also an antibody. These may be used singly or in combination of two or more.

The dextranase may include not only those dextranases that are obtained by known techniques from known dextranase-producing bacteria belonging to Genus Chetomium, Genus Penicillium, Genus Aspergillus, Genus Spikalia, Genus Bactobacillus, Genus Celluvibrio and the like, but also dextranases produced from other types of microorganisms, and are commercially available as those of Sankyo Co., Ltd. It will be noted that a dextranase is usually added in an amount of 1 to 100 units, preferably 10 to 50 units, per gram of a dentifrice composition.

The mutanase may include not only those mutanases that are obtained by known techniques from known dextranase-producing bacteria belonging, for example, to Genus Pseudomonas, Genus Trichoderma, Genus Streptomyces, Genus Aspergillus, Genus Flavobacterium, Genus Bacillus and the like, but also mutanases produced from other types of microorganisms. A mutanase is usually added in an amount of 10 to 10,000 units, preferably 20 to 5,000 units, per g of a dentifrice composition.

It will be noted that the amount of other types of enzymes may be appropriately selected, and such enzymes may be formulated in an effective amount of suppressing tartar formation and also in an effective amount of suppressing gingivitis.

Examples of the antiplasmin agents include tranexamic acid, ipsiron-aminocaproic acid, dihydrocholesterol and the like. The content of the antiplasmin agent is not critical and is usually in the range of 0.0001 to 2% (percent by weight herein and hereinafter), preferably 0.001 to 1%, and more preferably 0.01 to 0.5%, of the total composition.

For the anionic surface active agents, one or more anionic surface active agents ordinarily used in the field of dentifrice are used, including sodium alkyl sulfates such as sodium lauryl sulfate, sodium myristyl sulfate and the like, sodium N-acylsarcosines such as sodium lauroylsarcosine, sodium N-myristylsarcosine and the like, sodium dodecylbezenesulfonate, sodium hydrogenated coconut fatty acid monoglyceride monosulfate, sodium laurylsulfoacetate, N-acylglutaminates such as sodium N-palmitoylglutaminate and the like, sodium N-methyl-N-acyltaurines, sodium N-methyl-N-acylalanines, sodium α-olefinsulfonates and the like. It will be noted that from the standpoint of feeling upon use, sodium lauryl sulfate is preferred. The amount of the anionic surface active agent is generally 0.1 to 5%, preferably 0.5 to 3%, and more preferably 0.8 to 2%, of the total composition.

In the practice of the invention, a hydroxyethyl cellulose·dimethyldiallylammonium salt, which is a cationic polymer, is selectively used. The hydroxyethyl cellulose· dimethyldiallylammonium salt used herein means a cationic polymer obtained by graft-polymerizing a dimethyldiallylammonium salt to hydroxyethyl cellulose. The counter ion is a halogen ion such as a chlorine ion, a methylsulfate ion, or the like. The average molecular weight of the cationic polymer is not critical and preferably ranges 1,000 to 1,000,000. The content of nitrogen ranges 0.1 to 3%, preferably 0.5 to 2.5%. As the hydroxyethyl cellulose·dimethyldiallylammonium salt, Cellcoat L-200 and Cellcoat H-100 that are commercially available from Nippon NSC, Ltd. may be used. The counter ion of these salts consists of chlorine ion. If this cationic polymer is replaced by other type of cationic polymer, e.g. Leoguard KGP (Lion Corporation), inconvenience may be caused in that an aggregate mass is formed during the course of preparation, and thus, the problem to be solved by the invention cannot be solved satisfactorily.

The content of the cationic polymer generally ranges 0.001 to 2%, preferably 0.005 to 1%, and more preferably 0.01 to 0.5%, of the total composition. If the content is less than 0.001%, the retaining effect of an enzyme and an antiplasmin agent may not be shown satisfactorily. On the contrary, the content over 2% is unfavorable because of the development of unique offensive taste and offensive odor.

In the invention, one or more flavor components (A) selected from anethole, carvone, cineol, methyl salicylate, eugenol, ethyl butylate and cinnamic aldehyde and 1-menthol (B) are formulated in combination. As the flavor component (A), anethole is most preferred. Although the flavor components may be formulated as a pure substance, an essential oil containing a flavor component may also be used, e.g. with carvone, a spearmint oil may be formulated. With respect to 1-menthol used in combination with the flavor component, this may be formulated in the form of a pure substance, and an essential oil containing menthol, such as, for example, peppermint oil, Japanese mint oil or the like, may also be used.

The mixing ratio by weight between the total amount of the flavor components (A) and 1-menthol (B) is in the range of 1:9 to 8:2, preferably 2:8 to 7:3. The formulation within this range of the ratio permits the intraoral retention of an enzyme and an antiplasmin agent to be enhanced, and ensures effective prevention of syneresis of a preparation and leads to an improved feeling upon use, especially with respect to foaming or the like.

It will be noted that the amount of the flavor component (A) ranges 0.001 to 1.0%, preferably 0.005 to 0.5%.

So far as the effect of the invention is not impeded considerably, the composition of the invention may be further formulated with appropriate components ordinarily used in dentifrice compositions such as abrasives, binders, thickening agents, humectants, surface active agents, sweetening agents, flavors, colorants, preservatives, storage stabilizing agents, pH adjusting agents, effective ingredients and the like.

As the abrasive, inorganic abrasives including calcium phosphate compounds such as calcium hydrogen phosphate dihydrate and anhydride, calcium dihydrogen phosphate, calcium phosphate, calcium pyrophosphate and the like, calcium carbonate, calcium hydroxide, alumina, aluminium silicate, insoluble sodium metaphosphate, magnesium phosphate, magnesium carbonate, calcium sulfate, bentonite, zirconium silicate, titanium-bonded silicate and the like, and organic abrasives such as methyl polymethacrylate, crystaline cellulose and the like may be exemplified. The amount of the abrasive ranges 5 to 60%, preferably 8 to 50%, of the total composition.

As the binder, organic binders such as carrageenan, cellulose derivatives such as sodium carboxymethyl cellulose, methyl cellulose, hydroxyethyl cellulose and the like, gums such as xanthan gum, tragacanth gum, karaya gum, gum arabii and the like, polyvinyl alcohol, carboxyvinyl polymers such as crosslinking sodium polyacrylate, non-crosslinking sodium polyacrylate and the like, polyvinyl pyrrolidone and the like, and inorganic binders such as silica gel, aluminium silica gel, bee gum, laponite and the like may be exemplified. The amount of the binder usually ranges 0.2 to 2% of the total composition.

As the thickening agent and humectant, polyhydric alcohols such as sorbitol, glycerine, ethylene glycol, propylene glycol, 1,3-butyleen glycol, polyethylene glycol, polypropylene glycol, xylitol, maltitol and the like, and sugar alcohols may be exemplified.

Nonionic surface active agents, cationic surface active agents, and amphoteric surface active agents may be formulated as the surface active agent. Examples of the nonionic surface active agent include sugar alcohol fatty acid esters such as sorbitan fatty acid esters, polyoxyethylene sorbitan fatty acid esters, succharose fatty acid esters, lactitol fatty acid esters and the like, polyhydric alcohol fatty acid esters such as glycerine fatty acid esters, polyglycerine fatty acid esters, polyoxyethylene glycerine fatty acid esters, polyethylene glycol fatty acid esters and the like, ether-type surface active agents such as polyoxyethylene alkyl ethers, polyoxyethylene polyoxypropylene copolymers, polyoxyethylene alkylphenyl ethers, polyoxyethylene hardened castor oil and the like, fatty acid alkanolamides such as lauric acid diethanolamide, and the like. Examples of the cationic surface active agent include alkylammonium and alkylbenzylammonium salts, and the like. Examples of the amphoteric surface active agent include acetic acid betaine, imidazolium betaine, lecithin, and the like.

As the sweetening agent, sodium saccharine, stevioside, neosperidyl dihydrochalcone, glycyrrhizin, perillartine, p-methoxycinnamic aldehyde, aspartame, xylitol and the like may be exemplified.

As the flavor, flavors known in the field of dentifrice other than the flavor component (A) and 1-menthol (B) can be appropriately exemplified.

Examples of the colorants include red #2, red #3, red #225, yellow #4, yellow #5, yellow #205, blue #1, blue #2, blue #201, blue #204, green #3, titanium mica, titanium oxide and the like.

Examples of the preservatives include sodium benzoate, methylparaben, cetylpyridinium chloride, isopropylmethylphenol, sorbic acid potassium salt and the like.

Examples of the storage stabilizing agents include vitamin C, vitamin E, sodium sulfite, sodium pyrosulfite, sodium pyrohydrosulfite, butylhydroxytoluene, propyl gallate, butylhydroxyanisole and the like.

Examples of the pH adjuster include citric acid, malic acid, lactic acid, tartaric acid, acetic acid, phosphoric acid, pyrophosphoric acid, glycerophosphoric acid and salts thereof, and sodium hydroxide. The pH of the dentifrice of the invention is so adjusted as to be within a range of 5 to 9, preferably 6 to 8.

Examples of the effective ingredients include antimicrobial agents such as chlorhexidine, triclosan, cetylpyridium chloride, hinokitiol and the like, fluorine compounds such as sodium fluoride, stannous fluoride, sodium monofluorophosphate and the like, dental calculus preventive agents such as polyphosphoric acids and the like, gingivatightening agents such as sodium chloride and the like, various types of vitamins such as tocopherol acetate and the like.

The dentifrice composition of the invention exhibits not only an excellent intraoral retention-improving effect of an enzyme and an antiplasmin agent, but also is foamed well and exhibits excellent storage stability while suppressing syneresis, so that it can be utilized as a dentifrice useful for preventing dental caries and a poeriodental disease.

Experimental examples, examples and comparative examples are shown to more particularly described the invention. The invention should not be construed as limit the invention to the following examples.

### Experimental Example 1

Dentifrices containing the components indicated in Table 1 were prepared, respectively. 1 g of each dentifrice was diluted with distilled water by a factor of three, and an apatite disk having a diameter of 1 cm and a thickness of 0.5 cm was placed in the resulting suspension. After 3 minutes, the apatite disk was removed and rinsed with distilled water, after which the strength of the dextranase retained on the apatite disk was measured. The strength testing method was conducted in the following way.

10 mg of dextrane (a commercially available product) was dissolved a 0.1 mole phosphate buffer solution having a pH of 6 to provide a substrate solution. The apatite disk treated in a manner as set out above was placed in 1 ml of the substrate solution, followed by reaction at 35°C for 10 minutes. The reduction power of the resulting reducing sugar was measured by the Somogyi-Nelson method. According to a calibration curve using glucose, the amount (calculated as glucose) of the reducing sugar formed by the treatment at 35°C for 1 minute was calculated. More particularly, the strength of a dextranase capable of producing reducing sugar having reduction power corresponding to 1 µg of glucose by the treatment at 35°C for 1 minute was taken as one unit.

Next, these dentifrice compositions were evaluated by five panelers with respect to foaming thereof. Moreover, 90 g of the dentifrice was charged into a laminated tube (material for inner layer: polyethylene), and syneresis after storage at 40°C over 6 months was evaluated. The evaluation was made in the following way. The results are shown in Table 1.

It will be noted that Cellcoat L-200 (Nippon NSC, Ltd.) was used as hydroxyethyl cellulose·dimethyldiallyl-ammonium salt and Leoguard KGP was used as O-[2-hydroxy-3-(trimethylammonium)propyl]hydroxyethyl cellulose chloride.
Evaluation standards for foaming:
   ⓞ: foamed immediately after brushing
   ○: foamed almost immediately after brushing
   ×: foamed in a short time after brushing
Evaluation standards for syneresis:
   ⓞ: no syneresis observed
   ○: little syneresis observed
   ×: syneresis appreciably observed

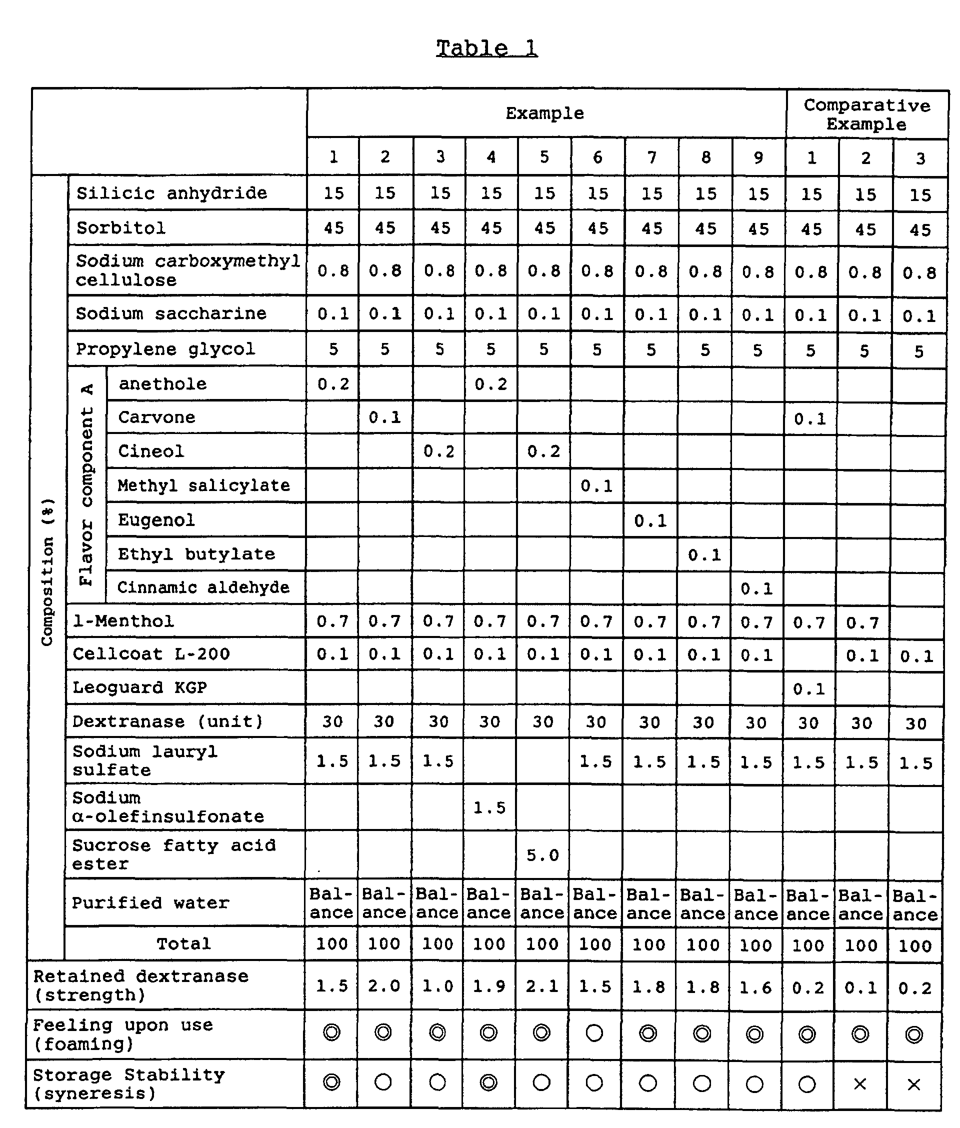

   From the results of Table 1, it was found that the dentifrices using the dextranase and the hydroxyethyl cellulose·dimethyldiallylammonium salt in combination were better in the retention of dextranase on the apatite disk than singly used compositions. In contrast, with O-[2-hydroxy-3-(trimethylammonium)propyl]hydroxyethyl cellulose chloride that is cationized cellulose different in type from hydroxyethyl cellulose·dimethyldiallylammonium salt, no improving effect of the dextranase retention was observed.
   Moreover, the results of Table 1 revealed that with dentifrices using, in combination, the flavor component selected from anethole, carvone, cineol, methyl salicylate, eugenol, ethyl butylate and cinnamic aldehyde, and 1-menthol, good results were obtained with respect to syneresis after storage at 40°C for 6 months. In contrast, with a composition using 1-menthol singly or a 1-menthol-free composition, no improving effect of syneresis was found. It will be noted that the degree of improvement of syneresis was better when anethole and 1-menthol were used in combination.

### Experimental Example 2

Dentifrices containing components indicated in Table 2 were prepared, respectively. 1 g of each dentifrice was diluted with and suspended in distilled water by a factor of three, followed by placing an apatite disk having a diameter of 1 cm and a thickness of 0.5 cm in the suspension. After 3 minutes, the apatite disk was removed and rinsed with distilled water, after which the strength of the mutanase retained on the apatite disk was measured. The strength testing method was carried out in the following way.

A 0.1 mole acetate buffer solution containing 3% of mutan was adjusted in pH to 5 thereby providing a substrate solution. The apatite disk treated in such a manner as stated above was placed in 1 ml of the substrate solution, followed by reaction at 35°C for 10 minutes. The reduction power of the resulting reducing sugar was measured by the Somogyi-Nelson method. According to a calibration curve using glucose, the amount (calculated as glucose) of the reducing sugar formed by the treatment at 35°C for 1 minute was calculated. More particularly, the strength of the mutanase capable of producing reducing sugar having reduction power corresponding to 1 µg of glucose by the treatment at 35°C for 1 minute was taken as one unit.

Next, these dentifrice compositions were evaluated by five panelers with respect to the foaming thereof. Moreover, 90 g of the dentifrice was charged into a laminated tube (material for inner layer: polyethylene), and syneresis after storage at 40°C over 6 months was evaluated in the same manner as set out hereinbefore. The results are shown in Table 2.

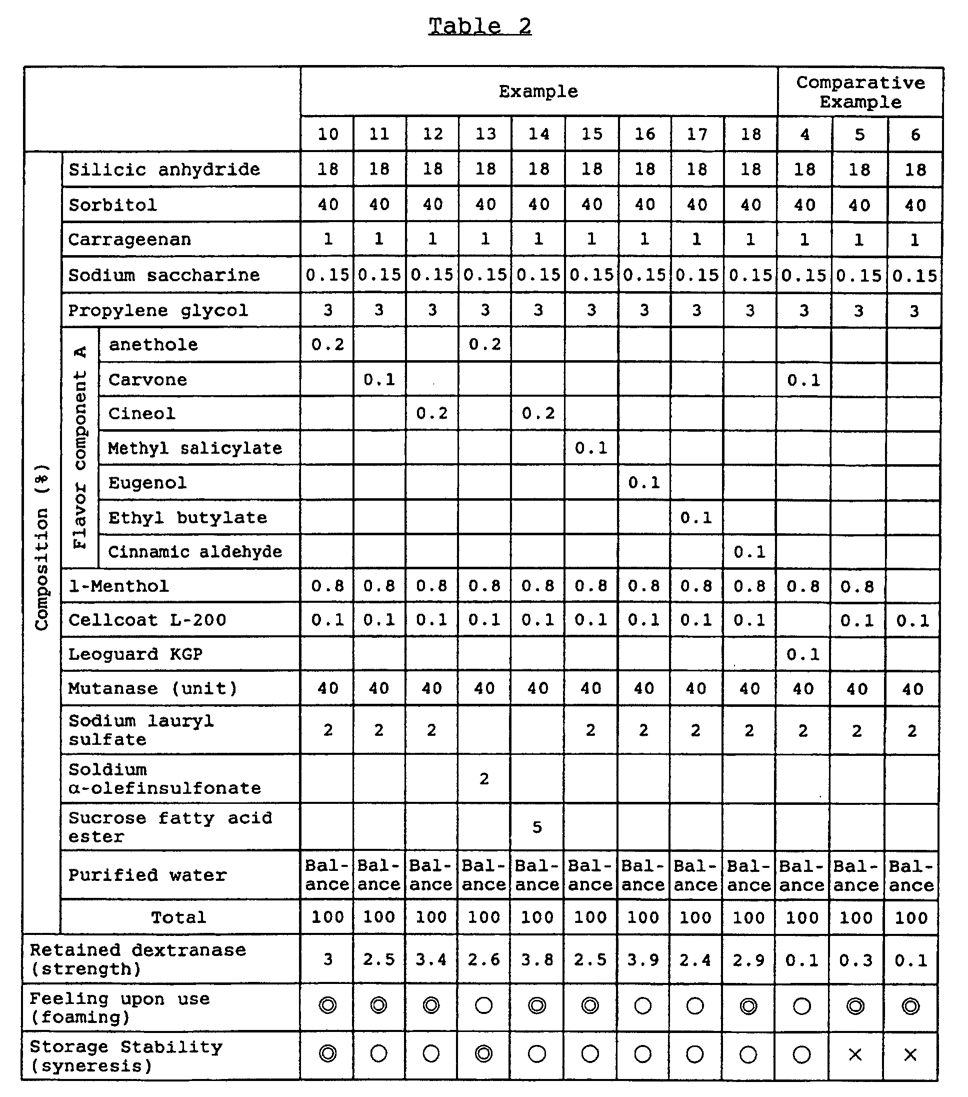

From the results of Table 2, it was found that the dentifrices using mutanase and hydroxyethyl cellulose dimethyldiallylammonium salt in combination were better in the retention of mutanase on the apatite disk than singly used compositions. In contrast, with O-[2-hydroxy-3-(trimethylammonium)propyl]hydroxyethyl cellulose chloride that is cationized cellulose different in type from hydroxyethyl cellulose·dimethyldiallylammonium salt, no improving effect of the mutanase retention was observed.

Moreover, the results of Table 2 revealed that with dentifrices using, in combination, the flavor component selected from anethole, carvone, cineol, methyl salicylate, eugenol, ethyl butylate and cinnamic aldehyde, and 1-menthol, good results were obtained with respect to syneresis after storage at 40°C for 6 months. In contrast, with a composition using 1-menthol singly or a 1-menthol-free composition, no improving effect of syneresis was found. It will be noted that the degree of improvement of syneresis was better when anethole and 1-menthol were used in combination.

### Experimental Example 3

Dentifrices containing the components indicated in Table 3 were prepared, respectively. A hydroxyapatite disk having a diameter of 1 cm and a thickness of 0.5 cm was immersed in a supernatant liquid of a 1:3 dilution of each dentifrice for 3 minutes, followed by washing three times with water. The concentration of tranexamic acid retained on the hydroxyapatite disk was measured.
Next, the foaming of these dentifrice compositions was evaluated by five panelers in the same manner as set out hereinbefore. Moreover, 90 g of each dentifrice was charged into a laminated tube (material for inner layer: polyethylene), and syneresis after storage at 40°C for 6 months were similarly evaluated. The results are shown in Table 3.

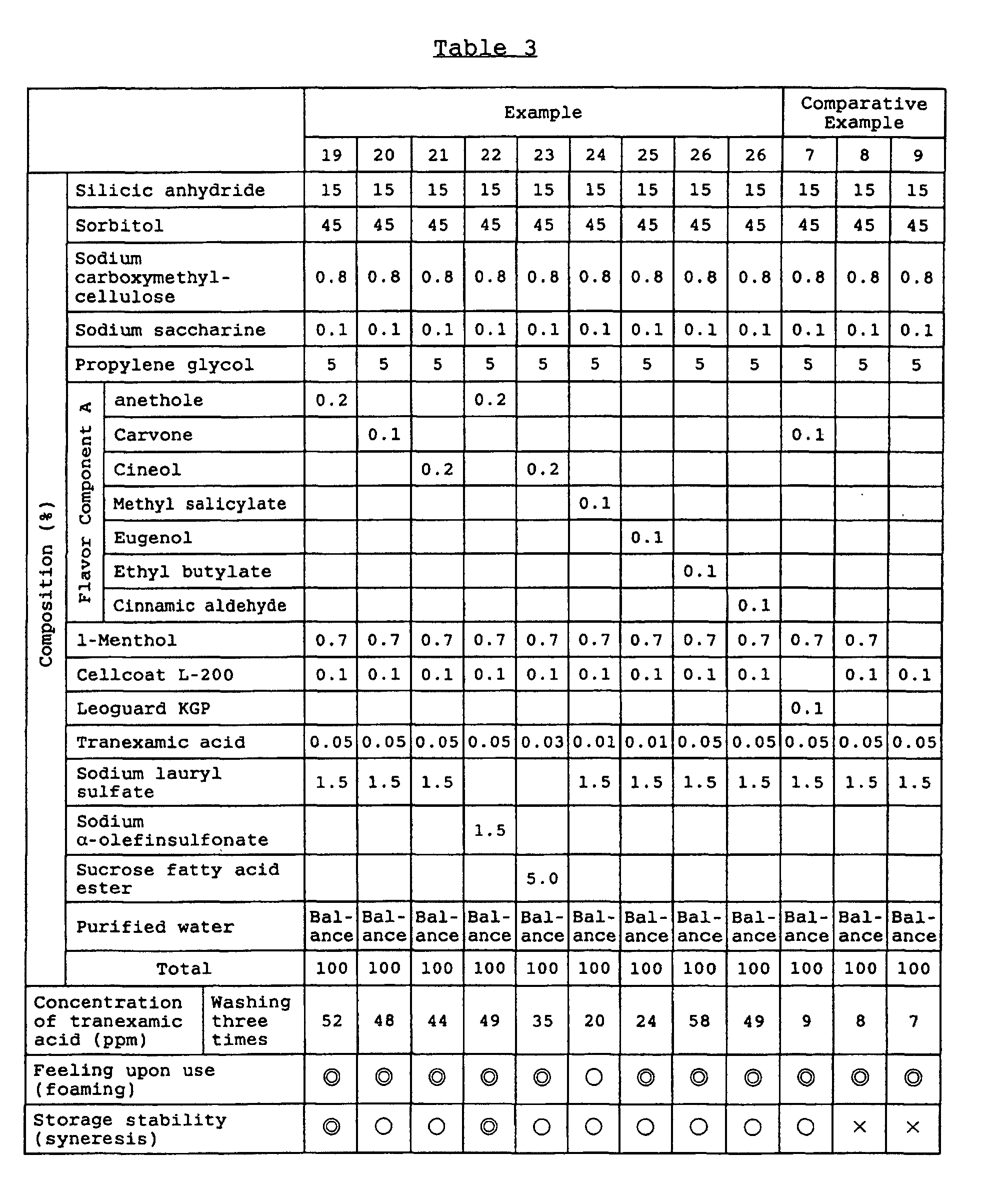

From the results of Table 3, it was found that the dentifrices using tranexamic acid and hydroxyethyl cellulose·dimethyldiallylammonium salt in combination were better in the retention of tranexamic acid on the apatite disk than singly used compositions. The hydroxyethyl cellulose·dimethyldiallylammonium salt was effective when its concentration was 0.01% or over, with an increasing amount of retention at 0.03% or over. In contrast, with O-[2-hydroxy-3-(trimethylammonium)propyl]hydroxyethyl cellulose chloride that is cationized cellulose different in type from the hydroxyethyl cellulose·dimethyldiallylammonium salt, no improving effect of the retention was observed.

Moreover, the results of Table 3 revealed that with dentifrices using, in combination, the flavor component selected from anethole, carvone, cineol, methyl salicylate, eugenol, ethyl butylate and cinnamic aldehyde, and l-mentol, good results were obtained with respect to syneresis after storage at 40°C for 6 months. In contrast, with a composition using l-menthol singly or a l-menthol-free composition, no improving effect of syneresis was found. It will be noted that the degree of improvement of syneresis was better when anethole and l-menthol were used in combination.

## Claims

1. A dentifrice composition comprising an enzyme and/or an antiplasmin agent, and an anionic surface active agent, **characterized by** further comprising a hydroxyethyl cellulose·dimethyldiallylaminonium salt, and one or more flavor components (A) selected from the group consisting of anethole, carvone, cineol, methyl salicylate, eugenol, ethyl butylate and cinnamic aldehyde and l-menthol (B) being contained at a ratio by weight of 1:9 to 8:2.

2. A dentifrice composition according to Claim 1, wherein said enzyme consists of a dextranase and/or a mutanase.

3. A dentifrice composition according to Claim 1, wherein said antiplasmin consists of tranexamic acid and/or a salt thereof.
